# EUROPÄISCHE PATENTSCHRIFT

(11) **EP 0 800 800 B1**
(45) Veröffentlichungstag und Bekanntmachung des Hinweises auf die Patenterteilung: **15.07.1998**
(21) Anmeldenummer: 96113858.3
(22) Anmeldetag: 29.08.1996
(51) Int. Cl.: A61F 2/06

(54) **Stent zur transluminalen Implantation in Hohlorgane**
Stent for transluminal implantation in a hollow organ
Stent pour implantation transluminale dans un organe creux

(30) Priorität: 10.04.1996 DE 19614160
(43) Veröffentlichungstag der Anmeldung: 15.10.1997
(60) Teilanmeldung: 97110029.2
(73) Patentinhaber: Variomed AG, 9496 Balzers (LI)
(72) Erfinder:
(74) Vertreter: Pellkofer, Dieter Dr.

(56) Entgegenhaltungen:
- EP-A- 0 541 443
- EP-A- 0 647 438
- EP-A- 0 679 372
- EP-A- 0 709 067
- EP-A- 0 709 068
- WO-A-95/26695

## Beschreibung

Die vorliegende Erfindung betrifft einen Stent zur transluminalen Implantation in Hohlorgane, insbesondere in Blutgefäße, Harnleiter, Speiseröhren oder Gallenwege, mit einem im wesentlichen röhrenförmigen Körper, der eine Vielzahl von Durchbrechungen aufweist, die jeweils von langgestreckten Begrenzungselementen begrenzt werden.

Stents dieser Art, die beispielsweise aus der EP-A-0 541 443, der EP-A-0 647 438 und der WO 95/26695 bekannt sind, werden zur Rekanalisation von krankhaft veränderten Hohlorganen eingesetzt. Dabei werden die Stents üblicherweise in einem komprimierten Zustand über einen Einführkatheter an die zu behandelnde Stelle innerhalb des Hohlorgans eingeführt, wo sie durch unterschiedliche Maßnahmen auf einen Durchmesser, der dem des gesunden Hohlorgans entspricht, exportiert werden können, so daß eine Stützwirkung des Hohlorgans, beispielsweise einer Gefäßwand erreicht wird.

Bei dem aus der EP-A-0 541 443 bekannten Stent handelt es sich um einen ballonexpandierten Stent, der im komprimierten Zustand auf einen speziellen Ballonkatheter montiert, bis zu der zu behandelnden Stelle des jeweiligen Hohlorgans eingeführt und dort durch Balloninsuflatation auf den gewünschten Durchmesser ausgedehnt wird. Der Stent besteht aus einer Aneinanderreihung von zylindrischen Abschnitten, von denen die außenliegenden Abschnitte starr und der innenliegende Abschnitt elastisch ausgebildet sind. Die Abschnitte werden jeweils durch Balloninsuflatation auf den gewünschten Durchmesser ausgedehnt, wobei durch die plastische Verformung der starren Abschnitte der Stent im expandierten Zustand seine Stabilität erhält, so daß eine ausreichende Stützwirkung des Hohlorgans erzielt wird. Durch die Aneinanderreihung von starren und flexiblen Abschnitten, die durch Schweißverbindungen miteinander verbunden sind, wird die insgesamt erforderliche Flexibilität des Stents gewährleistet. Um die Schweißverbindung zwischen den starren und den flexiblen Abschnitten zu ermöglichen, sind an den Enden der starren Abschnitte in Umfangsrichtungen Verbreiterungen vorgesehen, an die die Enden der flexiblen Abschnitte befestigbar sind. Diese Verbreiterungen besitzen in Längsrichtung eine relativ geringe Ausdehnung, so daß sie nicht als Detektionselemente offenbart sind und aufgrund ihrer geringen Ausdehnung in Längsrichtung auch nicht als solche wirksam wären.

Aus der WO 95/26695 ist ein selbstexpandierender Stent bekannt, der durch einen Haltedraht in seinem komprimierten Zustand gehalten und in diesem Zustand über einen Katheter an die zu behandelnde Stelle innerhalb des Hohlorgans eingeführt wird. Nach Entfernen des Haltedrahts dehnt sich der Stent durch die ihm eigene Spannung von selbst auf einen vorbestimmten Durchmesser im Hohlorgan aus, so daß auf diese Weise die Stützung der Wand des Hohlorgans erreicht wird. Darüber hinaus ist in der WO 95/26695 auch beschrieben, daß diese Stents auch mit Hilfe eines Ballonkatheters expandiert werden können.

Aus der nachveröffentlichten EP-A-0 709 068, die einen Stand der Technik gemäß Art. 54(3) EPU bildet, ist ein Stent bekannt, an dessen Enden konzentrisch ausgebildete, ringförmige Detektionselemente vorgesehen sind. Die wirksame Fläche dieser Detektionselemente ist jedoch relativ klein und kann aufgrund der konzentrischen Ausbildung auch nicht größer gewählt werden, da sich die in Umfangsrichtung nebeneinanderliegenden Detektionselemente gegenseitig stören wurden.

Zu der Gruppe der selbstexpandierenden Stents gehören auch Stent aus dem sogenannten "Memory-Metall" Nitinol (eingetragenes Warenzeichen). Nitinol ist eine Nickel-Titan-Legierung mit temperaturabhängigem Formverhalten. Wird beispielsweise einem Nitinoldraht eine bestimmte Form eingeprägt und anschließend der Draht über eine bestimmte "Memory-Temperatur" erhitzt, so gewinnt dieser Draht ein "Erinnerungsvermögen" für diese Form. Kühlt man anschließend den so behandelten Draht wieder unter seine Konversionstemperatur ab, die von der Legierung und der Wärmebehandlung abhängig ist, so wird er weich und leicht verformbar. Bei einer erneuten Erwärmung über die Konversionstemperatur nimmt der Draht automatisch die eingeprägte Form wieder an.

Ein Stent aus Nitinol ist beispielsweise in der EP-A-0 647 438 beschrieben. Der in dieser Druckschrift beschriebene Stent wird in seinem komprimierten Zustand an die gewünschte Stelle des Hohlorgans über einen Katheter eingeführt und weitet sich nach Entfernen des Katheters in seine expandierte Form selbsttätig aus. Der Stent ist als Reduzierstent zum Reduzieren des Durchmessers einer von Blut durchflossenen Ader ausgebildet, indem er in seinem Mittenbereich einen verringerten, frei durchfließbaren Bereich und in seinem radialen Außenbereich trombogene Fäden besitzt, an denen sich nach dem Einsetzen Blutgerinnsel festsetzen, wodurch der effektive Durchflußbereich reduziert wird.

Selbstexpandierende Stent der eingangs genannten Art werden beispielsweise dadurch erzeugt, daß mit einem Laser in die Wand eines röhrenförmigen Körpers mit geringem Durchmesser parallel zu dessen Längsachse verlaufende Schlitze geschnitten werden. Diese Schlitze sind in Umfangsrichtung versetzt zueinander angeordnet, so daß bei einer Expansion des röhrenförmigen Körpers, beispielsweise durch Balloninsuflatation oder durch Erwärmung im Falle eines Stents aus Memory-Metall, rautenförmige Durchbrechungen entstehen, deren Längsachsen ebenfalls parallel zur Längsachse des röhrenförmigen Körpers verlaufen.

Das zwischen den Durchbrechungen liegende Material der Wand des röhrenförmigen Körpers bildet dabei langgestreckte Begrenzungselemente, durch die die Durchbrechungen voneinander getrennt und begrenzt werden. Um eine gute Flexibilität des Stents zu gewährleisten, betragen sowohl die Breite als auch die Dicke der langgestreckten Begrenzungselemente circa 0,2 bis 0,25 mm, wobei Breite und Dicke ungefähr gleich groß gewählt werden müssen, da anderenfalls beim Expandieren des röhrenförmigen Körpers Verwindungen der Begrenzungselemente auftreten würden, welche zum Abknicken des Stents führen können.

Ein kritischer Punkt bei der Verwendung von Stents liegt darin, daß der Stent beim Einsetzen millimetergenau innerhalb des Hohlorgans positioniert werden muß, da nur bei einer exakten Positionierung an der krankhaft veränderten Stelle des Hohlorgans dessen vollständige Aufweitung erreicht werden kann. Darüber hinaus muß beispielsweise bei einer krankhaften Verengung im Bereich einer seitlichen Abzweigung des Hohlorgans eine Überdeckung des Abzweigungskanals durch den Stent vermieden werden, so daß auch in diesem Fall eine exakte Positionierung des Stents gewährleistet sein muß.

Weiterhin können Stents auch nach erfolgter korrekter Pösitionierung innerhalb des Hohlorgans zu wandern beginnen, so daß auch nach Einsetzen des Stents dessen Position innerhalb des Hohlorgans kontrollierbar sein muß.

Sowohl das Einsetzen des Stents als auch die Kontrolle der Position eines eingesetzten Stents wird unter Beobachtung am Röntgenschirm durchgeführt, auf dem der üblicherweise aus Metall bestehende Stent als heller Fleck sichtbar ist. Aufgrund der geringen Dicke und Breite der langgestreckten Begrenzungselemente wird jedoch durch den expandierten Stent nur ein relativ schwaches Bild am Röntgenschirm erzeugt, so daß sowohl die Position des teil- und vollständig expandierten Stents während des Einsetzens bzw. unmittelbar nach dem Einsetzen als auch die Position des eingesetzten Stents bei einer Nachkontrolle nur schlecht erkannt werden kann.

Darüber hinaus führt die Expansion des Stents üblicherweise zu einer Verkürzung des Stents in Längsrichtung und damit zu einer Verschiebung zumindest der Stentenden. Da das Ausmaß der Verkürzung von dem Ausmaß der Expansion und damit von dem nicht genau bekannten Durchmesser des Hohlorgans abhängt, wird die genaue Positionierung des Stents weiter erschwert.

Ein weiteres Problem der bekannten Stents liegt darin, daß beim Expandieren des Stents durch Aufweiten der Durchbrechungen die langgestreckten Begrenzungselemente insbesondere an den Verbindungsstellen zwischen zwei in Längsrichtung des Stents benachbart angeordneten Durchbrechungen so stark belastet werden, daß an diesen Steilen Bruchstellen entstehen können. Die an diesen Bruchstellen entstehenden spitzen Enden ragen insbesondere bei einer Verbiegung des Stents, wie sie beispielsweise bei einer Kurvenimplantation auftritt, am äußeren Kurvenradius aus der Wand des Stents hinaus und am inneren Kurvenradius in den Innenbereich des Stents hinein. Dies hat zur Folge, daß Verletzungen der Wand des Hohlorgans und des Ballons eines verwendeten Ballonkatheters auftreten können. Dies sind Komplikationen, die in der Praxis nicht hingenommen werden dürfen.

Auch bei eingesetzten Stents können Bruchstellen dieser Art auftreten, da beispielseweise durch Bewegungen des Patienten oder durch Bewegungen der inneren Organe der Stent dauernden Wechselbelastungen ausgesetzt ist, die letzlich zu den beöschriebenen Bruchstellen führen können. Auch diese bei eingesetzten Stents auftretetenden Bruchstellen können in der Praxis nicht hingenommen werden.

Der vorliegenden Erfindung liegt daher die Aufgabe zugrunde, einen Stent der eingangs genannten Art zu schaffen, dessen Position sowohl während des Einsetzens als auch nach dem Einsetzen exakt bestimmbar ist.

Diese Aufgabe wird nach der Erfindung ausgehend von einem Stent der eingangs genannten Art durch die kennzeichnenden Merkmale des Anspruchs 1 gelöst.

Durch das Zusammenfassen von zwei benachbart angeordneten Begrenzungselementen zu einem einheitlichen Detektionselement mit vergrößerter Breite werden flächige Materialbereiche geschaffen, die am Röntgenschirm besser erkennbar sind als die einzelnen, dünn ausgebildeten langgestreckten Begrenzungselemente. Dadurch kann ein erfindungsgemäß ausgebildeter Stent im expandierten Zustand am Röntgenschirm besser erkannt und damit exakter positioniert werden als ein Stent bekannter Bauart. Darüber hinaus kann die Position eines eingesetzten erfindungsgemäßen Stents anhand der verbreiterten Detektionselemente exakt bestimmt werden. Durch die Anordnung der Detektionselemente am Ende des Stents kann, auch bei einer durch die Expansion verursachten Verkürzung des Stents, jeweils die exakte Position ermittelt werden.

Ein weiterer Vorteil eines erfindungsgemäß ausgebildeten Stents liegt darin, daß durch die verbreiterten Detektionselemente der auf die Innenwände des Hohlorgans wirkende Anpreßdruck verringert wird, so daß eine verminderte Reibung bzw. Reaktion der Hohlraumwand erzielt wird. Dies gilt insbesondere deshalb, da die Detektionselemente am Ende des Stents vorgesehen sind, da ohne verbreiterte Detektionselemente in diesem Fall die Stentenden durch die spitzen Enden der Begrenzungselemente gebildet werden, so daß insbesondere im Bereich der Stentenden eine große Reaktion der Hohlraumwand hervorgerufen wird.

Nach einer vorteilhaften Ausführungsform der Erfindung bilden im wesentlichen über den gesamten Umfangsbereich des Stents jeweils zwei benachbart angeordnete Begrenzungselemente ein Detektionselement, wobei die Breite des Detektionselements insbesondere im wesentlichen gleich der Breite zweier nebeneinander liegender Begrenzungselemente ist. Auf diese Weise werden über den gesamten Umfang des Stents Detektionselemente erzeugt, so daß unabhängig von der Lage des Stents im Hohlorgan aus jeder Beobachtungsrichtung zumindest ein Detektionselement im wesentlichen in seiner vollen Breite zu erkennen ist.

Nach einer weiteren vorteilhaften Ausführungsform der Erfindung bilden in Umfangsrichtung alternierend jeweils zwei benachbart angeordnete Begrenzungselemente ein Detektionselement, während die darauffolgenden zwei Begrenzungselemente nicht über das Ende der von ihnen begrenzten Durchbrechung hinaus verlängert sind, wobei die Breite des Detektionselements insbesondere im wesentlichen gleich der Breite von vier nebeneinander liegenden Begrenzungselementen ist. Dadurch, daß jeweils zwei benachbart angeordnete Begrenzungselemente nicht über das Ende der von ihnen begrenzten Durchbrechung hinaus verlängert sind, können die zwei sich in Umfangsrichtung anschließenden benachbart angeordneten Begrenzungselemente ein vierfach verbreitertes Detektionselement bilden, so daß der Stent noch besser am Röntgenschirm beobachtbar ist.

Grundsätzlich können auch noch mehr Begrenzungselemente nicht über das Ende der von ihnen begrenzten Durchbrechung hinaus verlängert sein, so daß die Detektionselemente noch breiter ausgebildet sein können. Zwischen den Detektionselementen können im komprimierten Zustand des Stents auch Abstände in Umfangsrichtung des Stents vorhanden sein, so daß die Breite der Detektionselemente grundsätzlich beliebig gewählt werden kann, solange sie größer als die Breite eines einzelnen Begrenzungselementes ist.

Bevorzugt sind die Detektionselemente an beiden Enden des Stents vorgesehen, so daß bei einer Kontrolle der Stentposition über den Röntgenschirm beide Enden des Stents einwandfrei erkennbar sind. Auf diese Weise ist es möglich, die Enden des Stents am Röntgenschirm sicher zu erkennen und somit eine genaue Aussage zu treffen, über welchen Abschnitt des Hohlorgans sich der eingesetzte Stent erstreckt.

Nach einer weiteren vorteilhaften Ausführungsform der Erfindung sind die die Expansion gewährleistenden Durchbrechungen sowohl im komprimierten als auch im expandierten Zustand als in Längsrichtung und in Umfangsrichtung des Stents ausgedehnte, flächige Durchbrechungen ausgebildet, wobei im Bereich zwischen zwei in Längsrichtung des Stents benachbart angeordneten Durchbrechungen verstärkt ausgebildete Verbindungsstellen vorgesehen sind.

Diese Ausführungsform trägt der Problematik Rechnung, daß zur Erhöhung der Stabilität eines Stents nicht lediglich die schlitzförmigen Durchbrechungen in größeren Abständen voneinander erzeugt werden können, so daß die zwischen benachbart angeordneten Durchbrechungen vorhandenen Materialbereiche breiter ausgebildet wären, da Breite und Dicke der zwischen den Durchbrechungen vorhandenen Begrenzungselemente ungefähr gleich groß sein müssen. Würde man einfach die Abstände zwischen den schlitzförmigen Durchbrechungen vergrößern, so würde das Verhältnis von Breite zu Dicke der zwischen den Durchbrechungen vorhandenen Begrenzungselemente aus dem Gleichgewicht geraten so daß beim Expandieren des Stents bzw. durch die wechselnde Belastung beim eingesetzten Stent Verwindungen der Begrenzungselemente auftreten würden, die zum Abknicken des Stents führen können.

Durch die bevorzugte Lösung, die Durchbrechungen auch im komprimierten Zustand nicht nur als Schlitze, sondern flächig auszubilden und gleichzeitig die zwischen zwei in Längsrichtung des Stents angeordneten Durchbrechungen ausgebildeten Verbindungsstellen zu verstärken, wird erreicht, daß die bei der Expansion bzw. durch die Wechselbelastungen besonders beanspruchten Stellen stabiler ausgebildet sind und gleichzeitig Breite und Dicke der zwischen den Durchbrechungen vorhandenen Begrenzungselemente im wesentlichen gleich groß gewählt werden können. Es wird somit eine spannungsoptimierte Konstruktion geschaffen, die die für einen Stent erforderliche Stabilität und Flexibilität in optimaler Weise vereint.

Nach einer weiteren vorteilhaften Ausführungsform der Erfindung sind die verstärkt ausgebildeten Verbindungsstellen durch sich in Umfangsrichtung des Stents erstreckende Verbreiterungen der Begrenzungselemente gebildet. Auf diese Weise ist eine besonders einfache und kostengünstige Herstellung eines erfindungsgemäß ausgebildeten Stents möglich, da beispielsweise beim Erzeugen der Ausnehmungen durch Schneiden mit einem Laserstrahl entlang einer beispielsweise computergesteuerten Schnittlinie gleichzeitig die Verbreiterungen erzeugt werden können, so daß durch die Form der Durchbrechungen gleichzeitig die Form der Verbreiterungen der Begrenzungselemente gesteuert werden kann. Die Verbreiterungen der Begrenzungselemente können dabei beispielsweise dadurch erzeugt werden, daß die Enden der von den Begrenzungselementen begrenzten flächigen Durchbrechungen verjüngt ausgebildet sind oder daß erweiterte Bereiche der Begrenzungselemente in die in Umfangsrichtung benachbart angeordneten Durchbrechungen hineinragen.

Weitere vorteilhafte Ausführungsformen sind in den Unteransprüchen angegeben.

Die Erfindung wird nachfolgend anhand eines Ausführungsbeispiels unter Bezugnahme auf die Zeichnungen näher beschrieben; in diesen zeigen:
- Fig. 1: ein verkürztes, flächig dargestelltes Schnittmuster zur Erzeugung der Durchbrechungen und der Detektionselemente für einen erfindungsgemäßen Stent,
- Fig. 2: eine Detailansicht nach Fig. 1,
- Fig. 3: eine Detailansicht ähnlich der Fig. 2 einer weiteren Ausführungsform eines erfindungsgemäß ausgebildeten Stents,
- Fig. 4: ein verkürztes, flächig dargestelltes Schnittmuster zur Erzeugung der Durchbrechungen und der Detektionselemente für eine weitere Ausführungsform eines erfindungsgemäß ausgebildeten Stents,
- Fig. 5: eine Detailansicht nach Fig. 4,
- Fig. 6: eine Detailansicht nach Fig. 5 des Stents im expandierten Zustand,
- Fig. 7: eine Detailansicht ähnlich Fig. 6 einer weiteren Ausführungsform eines erfindungsgemäß ausgebildeten Stents und
- Fig. 8: eine schematische Darstellunge der Seitenansicht eines erfindungsgemäß ausgebildeten Stents.

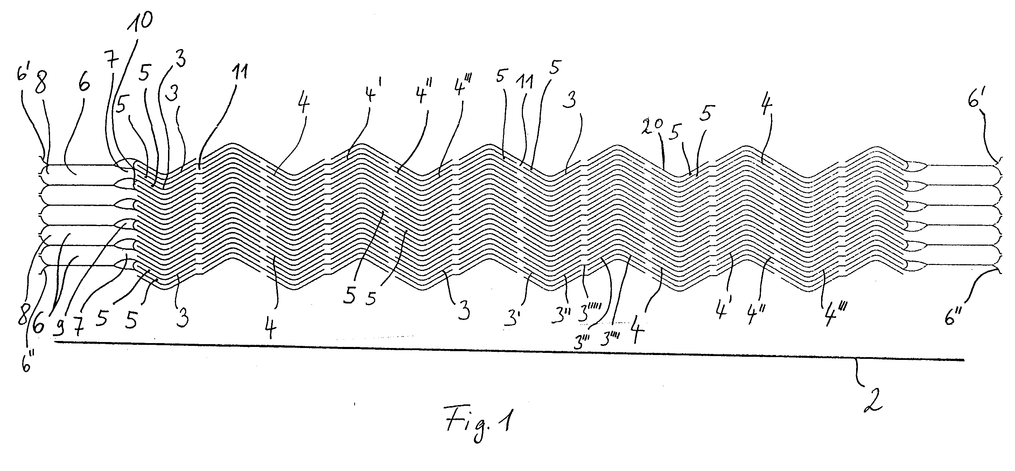

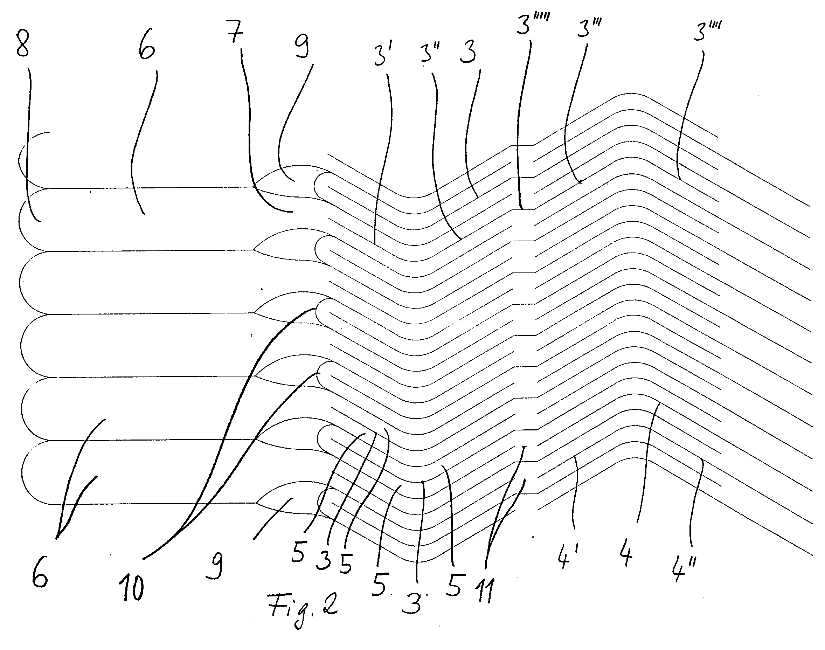

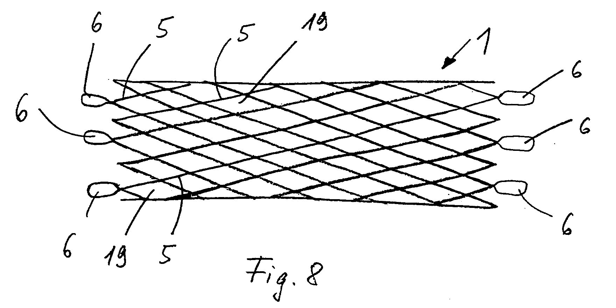

Fig. 1 und 2 zeigen ein Schnittmuster, wie es beispielsweise mittels eines Lasers in die Wand eines röhrenförmigen Körpers 1 (siehe Fig. 8) zur Herstellung eines erfindunsgemäß ausgebildeten Stents geschnitten wird. Zur Verdeutlichung der Lage der Schnitte innerhalb der Wand des röhrenförmigen Körpers 1 ist in Fig. 1 der Verlauf der Längsachse des röhrenförmigen Körpers 1 mit dem Bezugszeichen 2 versehen.

Durch den Schneidvorgang entstehen in der Wand des röhrenförmigen Körpers 1 Durchbrechungen 3, 4, die im komprimierten Zustand die Form der in Fig. 1 dargestellten schlitzförmigen Öffnungen besitzen.

Die schlitzförmigen Öffnungen 4 besitzen drei Abschnitte 4', 4'' und 4''', die jeweils schräg zur Längsachse 2 des röhrenförmigen Körpers 1 angeordnet sind und zusammen jeweils Z-förmige schlitzförmige Öffnungen 4 bilden. Die schlitzförmigen Öffnungen 3 besitzen vier Abschnitte 3', 3'', 3''' und 3'''', die jeweils schräg zur Längsachse 2 des röhrenförmigen Körpers angeordnet sind, wobei jeweils die Abschnitte 3' und 3'' bzw. 3''' und 3'''' V-förmige schlitzförmige Öffnungen bilden, die über einen fünften, parallel zur Längsachse 2 verlaufenden Abschnitt 3''''' miteinander verbunden sind.

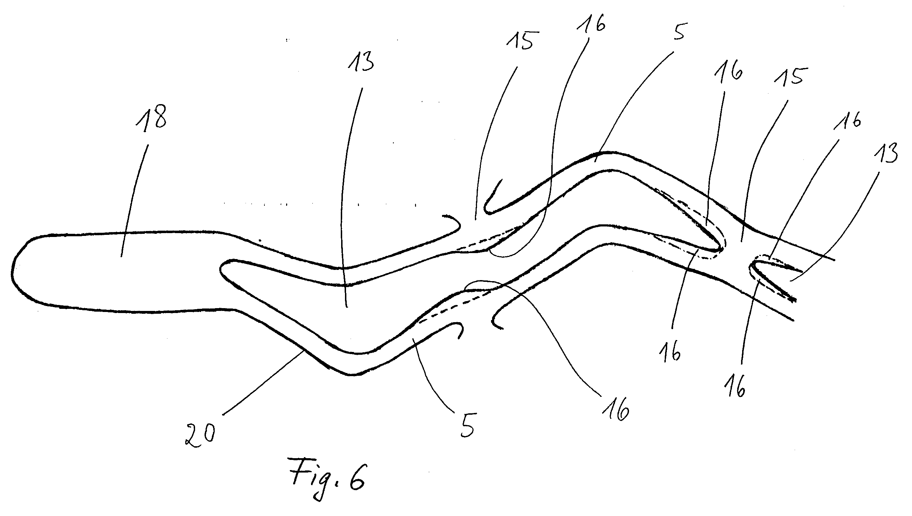

Das zwischen den Durchbrechungen 3, 4 liegende Material der Wand des röhrenförmigen Körpers 1 bildet jeweils Begrenzungselemente 5 für die Durchbrechungen 3, 4. Dabei bilden jeweils zwei in Umfangsrichtung des Stents benachbart angeordnete Begrenzungselemente 5 ein Umrandungselement 20 für eine Durchbrechung 3, 4, wie es besonders deutlich aus Fig. 6 ersichtlich ist.

An beiden Enden des Stents sind jeweils in Umfangsrichtung alternierend zwei benachbart angeordnete Begrenzungselemente 5 über das Ende der von ihnen begrenzten Durchbrechung 3 hinaus verlängert ausgebildet und zu einem einheitlichen, zungenförmigen Detektionselement 6 verbunden. Das Detektionselement 6 besitzt an seinem mit den Begrenzungselementen 5 verbundenen Ende 7 circa die doppelte Breite eines Begrenzungselementes 5 und verbreitert sich in Richtung seines freien Endes 8 auf circa die vierfache Breite eines Begrenzungselementes 5, so daß in Umfangsrichtung des Stents benachbart angeordnete Detektionselemente 6 über den Großteil ihrer Länge aneinander anliegen.

Zwischen den Enden 7 zweier benachbarter Detektionselemente 6 sind Ausnehmungen 9 ausgebildet, in die die Enden 10 von jeweils zwei benachbart angeordneten Begrenzungselementen 5 münden, die nicht über das Ende der von ihnen begrenzten Durchbrechung 3 hinaus verlängert sind. Grundsätzlich können die Enden 10 der Begrenzungselemente 5 jedoch auch direkt an den Seitenkanten der Enden 7 der Detektionselemente 6 anliegen, so daß keine Ausnehmungen 9 vorhanden sind.

Jeweils zwei in Umfangsrichtung des Stents benachbart angeordnete Begrenzungselemente 5, die nicht dieselbe Durchbrechung 3, 4 begrenzen, sind über Verbindungsstellen 11 miteinander verbunden. Die durch das in Fig. 1 dargestellte Schnittmuster erzeugten Durchbrechungen 3, 4 sowie die Detektionselemente 6 sind gleichmäßig über den gesamten Umfang des röhrenförmigen Körpers 1 verteilt, so daß beispielsweise die in Fig. 1 am oberen und unteren Rand jeweils zur Hälfte dargestellten Detektionselemente 6' und 6'' die beiden Hälften ein und derselben Detektionselemente 6 bilden.

Je nach Länge des röhrenförmigen Körpers 1 können mehr oder weniger Begrenzungselemente 5 und Durchbrechungen 3, 4 entlang der Längsachse 2 des röhrenförmigen Körpers 1 verteilt sein als in Fig. 1 dargestellt sind. Entsprechend kann je nach Umfang des röhrenförmigen Körpers 1 die Anzahl der Begrenzungselemente 5, der Durchbrechungen 3, 4 sowie der Detektionselemente 6 entlang des Umfangs des röhrenförmigen Körpers 1 variieren.

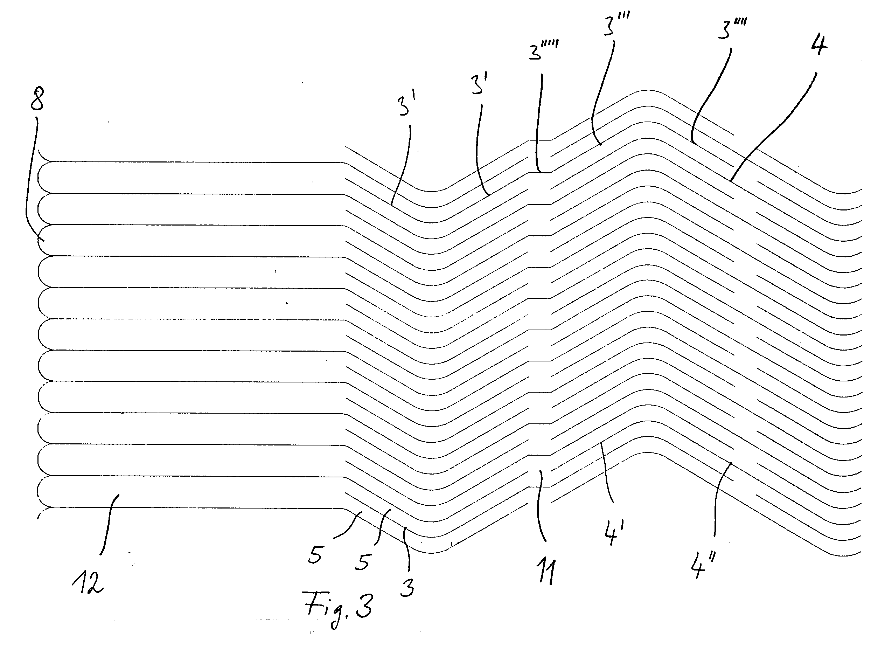

Bei der in Fig. 3 dargestellten Ausführungsform ist jedes der am Ende des Stents gelegenen Begrenzungselemente 5 über das Ende der von ihm begrenzten Durchbrechung 3 hinaus verlängert ausgebildet, so daß jeweils zwei in Umfangsrichtung benachbart angeordnete Begrenzungselemente 5 Detektionselemente 12 bilden, welche im wesentlichen doppelt so breit ausgebildet sind wie ein Begrenzungselement 5. Der Stent nach Fig. 3 umfaßt somit mehr Detektionselemente als der Stent nach den Fig. 1 und 2, wobei die Detektionselemente 12 eine geringere Breite aufweisen als die Detektionselemente 6 nach den Fig. 1 und 2, jedoch einen dem Radius des expandierten Stents besser angepaßten Krümmungsradius besitzen.

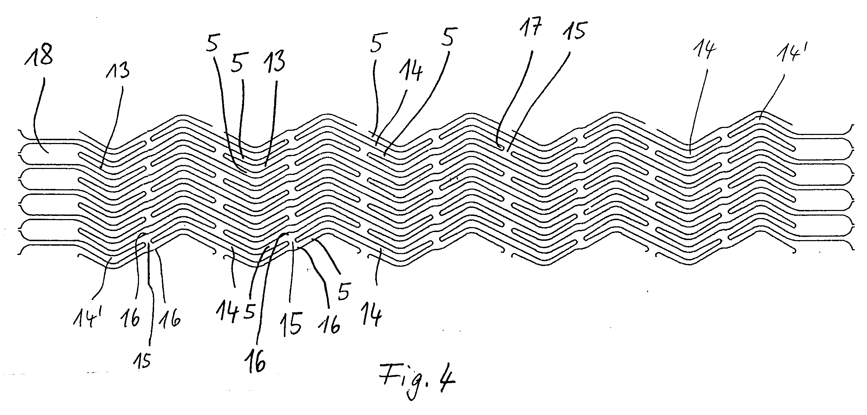

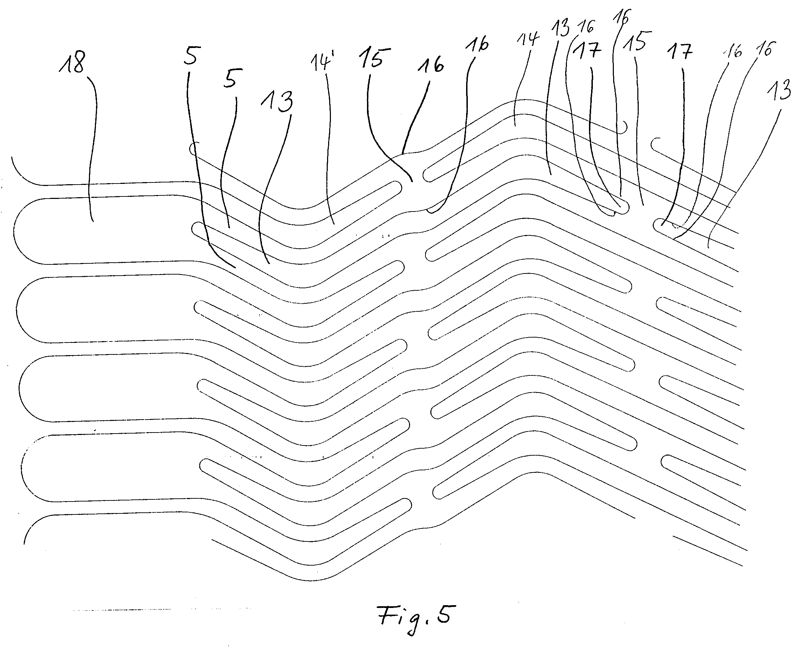

Bei dem Ausführungsbeispiel nach den Fig. 4 und 5 sind die Durchbrechungen nicht als schlitzförmige Öffnungen sondern als flächige Durchbrechungen 13, 14 ausgebildet. Wie nach den Ausführungsbeispielen gemäß den Fig. 1 bis 3 sind die Durchbrechungen 13, 14 von jeweils zwei Begrenzungselementen 5 umrandet, welche Umrandungselemente 20 für die Durchbrechungen 13, 14 bilden.

Zwischen jeweils zwei in Längsrichtung des Stents benachbart angeordneten Durchbrechungen 14 sowie zwischen den zu den Enden des Stents hin offenen Durchbrechungen 14' und den in Längsrichtung benachbarten Durchbrechungen 14 sind verstärkt ausgebildete Verbindungsstellen 15 vorgesehen, wobei die Verstärkungen als Verbreiterungen 16 der jeweiligen Enden der die Durchbrechungen 14, 14' begrenzenden Begrenzungselemente 5 ausgebildet sind. Die Kontur der Verbreiterungen 16 kann dabei unsymmetrisch zwischen den Durchbrechungen 14, 14' verlaufen, wie es beispielsweise in Fig. 5 dargestellt ist. Die Kontur kann jedoch auch symmetrisch ausgebildet sein, so daß die jeweils aneinanderstoßenden Enden zweier Begrenzungselemente 5 im wesentlichen spiegelsymmetrisch geformt sind.

Dadurch, daß die Verbreiterungen 16 im wesentlichen im Bereich zwischen den beiden Durchbrechungen 14 bzw. 14 und 14' vorgesehen sind, in dem beispielsweise beim Expandieren des Stents die größten Belastungen auf die Begrenzungselemente 5 wirken, können über die restliche Ausdehnung der Begrenzungselemente 5 deren Dicke und Breite ungefähr gleich groß gewählt werden, so daß beim Expandieren des Stents keine Verkippung oder Torsion der Begrenzungselemente 5 auftritt. Durch die Verbreiterungen 16 an den Verbindungsstellen zwischen den Enden zweier benachbarter Durchbrechungen 14 bzw. 14 und 14' wird gewährleistet, daß die Begrenzungselemente 5 im Bereich der Verbindungsstellen 15 so verstärkt ausgebildet sind, daß ein Bruch der Begrenzungselemente 5 beim Aufweiten der Durchbrechungen 14 zuverlässig verhindert wird.

Eine Verstärkung der Verbindungsstellen 15 kann zusätzlich oder allein auch dadurch erreicht werden, daß die Durchbrechungen 13 an ihren Enden 17 verjüngt verlaufend ausgebildet sind, wie es den Fig. 4 und 5 zu entnehmen ist, so daß die Begrenzungselemente 5 in Richtung zu den Verbindungsstellen 15 hin verbreitert ausgebildet sind. Auf diese Weise werden ebenfalls die zwischen zwei in Längsrichtung benachbart angeordneten Durchbrechungen 13 vorgesehenen Verbindungsstellen 15 so verstärkt ausgebildet, daß ein Bruch der Begrenzungselemente 5 beim Aufweiten der Durchbrechungen 13 verhindert wird.

Die an den Enden des Stents angeordneten Begrenzungselemente 5 sind wie bei dem Ausführungsbeispiel nach Fig. 3 jeweils über das Ende der von ihnen begrenzten Durchbrechung 13 hinaus verlängert ausgebildet, wobei jeweils zwei der verlängerten Abschnitte zu einem Detektionselement 18 verbunden sind, dessen Breite im wesentlichen der Breite von zwei Begrenzungselementen 5 plus der Breite der Durchbrechung 13 entspricht. Grundsätzlich können jedoch auch, wie beim Ausführungsbeispiel nach den Fig. 1 und 2, in Umfangsrichtung jeweils alternierend zwei benachbart angeordnete Begrenzungselemente 5 ein Detektionselement 18 bilden, während jeweils die zwei benachbart angeordneten Begrenzungselemente 5 nicht über das Ende der von ihnen begrenzten Durchbrechung 13 hinaus verlängert sind.

Fig. 6 zeigt eine Durchbrechung 13 nach dem Ausführungsbeispiel nach den Fig. 4 und 5 im aufgeweitetem Zustand, in dem eine Vergrößerung des Durchmessers des röhrenförmigen Körpers 1 erreicht wird. Weiterhin ist an den Verbindungsstellen 15 der Verlauf der Begrenzungselemente 5 ohne Verstärkung gestrichelt eingezeichnet, so daß der Unterschied zu einem Stent ohne erfindungsgemäß verstärkte Begrenzungselemente 5 und damit die verbesserte Belastbarkeit beim Verbiegen der Begrenzungselemente 5 erkennbar ist.

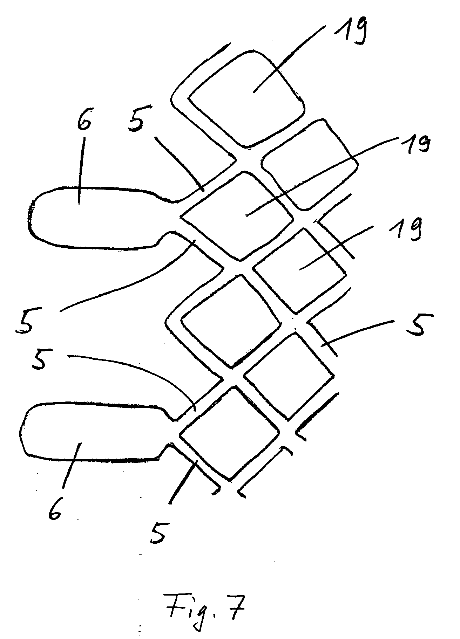

Die in Fig. 7 dargestellte Ausführungsform eines erfindungsgemäß ausgebildeten Stents umfaßt rautenförmige Durchbrechungen 19, die beim Aufweiten von parallel zur Längsachse 2 des Stents im komprimierten Zustand geschnittenen Schlitzen entstehen. Auch bei dieser Ausführungsform sind alternierend jeweils zwei im Endbereich des Stents angeordnete Begrenzungselemente 5 über das Ende der Öffnungen 19 hinaus verlaufend ausgebildet und zu einem blattförmigen Detektionselement 6 zusammengefaßt.

Aus der in Fig. 8 dargestellten Seitenansicht eines erfindungsgemäß ausgebildeten Stents ist besonders gut die Anordnung der Detektionselemente 6 an beiden Enden des röhrenförmigen Körpers 1 zu erkennen.

Der erfindungsgemäß ausgebildete Stent wird bevorzugt wie folgt hergestellt, vorbereitet und verwendet:

In die Wand eines aus Memory-Metall bestehenden röhrenförmigen Körpers 1 werden mit einem Laser eines der in den Fig. 1, 3 oder 4 dargestellten Schnittmuster bzw. parallel zur Längsachse 2 des Stents verlaufende, versetzt zueinander angeordnete Schlitze und damit die Durchbrechungen 3, 4 bzw. 13, 14 bzw. 19 geschnitten. Der Durchmesser des röhrenförmigen Körpers 1 wird dabei so gewählt, daß er dem zum Implantieren benötigten komprimierten Zustand des Stents entspricht.

Gleichzeitig mit den Durchbrechungen 3, 4 bzw. 13, 14 bzw. 19 werden an den Enden des röhrenförmigen Körpers 1 die blattförmigen Detektionselemente 6, 12, 18 erzeugt, welche jeweils als Verlängerungen von zwei in Umfangsrichtung des röhrenförmigen Körpers 1 benachbart angeordneten Begrenzungselementen 5 ausgebildet sind und abgerundete freie Enden 7 besitzen.

Nachdem das in den Fig. 1, 3 bzw. 4 dargestellte Schnittmuster bzw. die parallelen Schlitze über die gesamte Länge und den gesamten Umfang des röhrenförmigen Körpers 1 eingeschnitten wurden, wird der röhrenförmige Körper 1 auf eine Expansionsachse aufgezogen, deren Durchmesser dem im eingesetzten, expandierten Zustand benötigten Durchmesser des Stents entspricht. Dadurch werden zum einen die Öffnungen 3, 4 bzw. 13, 14 bzw. 19 ausgeweitet, wie es beispielsweise in den Fig. 6 und 7 dargestellt ist, und zum anderen wird der Abstand der in Umfangsrichtung benachbart angeordneten Detektionselemente 6, 12, 18 zueinander vergrößert. Anschließend wird durch Erhitzen des röhrenförmigen Körpers 1 über die Memory-Temperatur hinaus die entstandene Form in dem Material eingeprägt.

Nach Abkühlen des Stents unter die Konversionstemperatur kann der Stent wieder auf seinen dem komprimierten Zustand entsprechenden Ausgangsdurchmesser zusammengedrückt werden und mit einer elastischen Ummantelung, die beispielsweise aus Nylon (eingetragenes Warenzeichen), Polyethylen, Polyamid oder Polyurethanelastomeren besteht, überzogen oder in einen speziellen Katheter eingesetzt werden.

Der komprimierte Stent wird über einen Einführkatheter an die gewünschte Stelle innerhalb des Hohlorgans positioniert, wobei die Position des Stents über einen Röntgenschirm beobachtet wird. Eine Expansion des Stents vor vollendeter Positionierung wird dabei beispielsweise durch die elastische Ummantelung oder den speziellen Katheter verhindert. Durch Abstreifen der Ummantelung oder des Katheters über einen Teilbereich des röhrenförmigen Körpers 1 nimmt dieser in diesem Teilbereich aufgrund der über der Konversionstemperatur liegenden Körpertemperatur seine gespeicherte, expandierte Form an. Die durch die Expansion auftretende Verkürzung bewirkt dabei eine Verschiebung des freigelegten, expandierten Endes des röhrenförmigen Körpers 1.

Der Wandbereich zwischen den beiden Enden des Stents ist im expandierten Zustand des röhrenförmigen Körpers 1 unter dem Röntgenschirm nur schlecht erkennbar, da dieser Bereich nur durch die auseinandergezogenen, circa 0,2 mm breiten Begrenzungselemente 5 gebildet wird und daher kein ausreichendes Abbild auf dem Röntgenschirm erzeugt.

Da die Detektionselemente 6, 12, 18 jedoch deutlich breiter als die Begrenzungselemente 5 ausgebildet sind, sind die Enden des Stents auch im expandierten Zustand am Röntgenschirm deutlich zu erkennen, so daß die durch die Verkürzung auftretende Verschiebung der Enden des Stents bei Einsetzen in das Hohlorgan erkannt und der Stent korrekt positioniert werden kann.

Nachdem der Stent vollständig eingesetzt worden ist, kann seine Position anhand der an beiden Enden vorgesehenen Detektionselemente nochmals überprüft werden, so daß auch ein Wandern des Stents nach erfolgter, korrekter Positionierung weiter am Röntgenschirm erkennbar bleibt.

Außer durch die beschriebene Ausführungsform aus Memory-Metall können die Vorteile eines erfindungsgemäß ausgebildeten Stents auch bei Verwendung anderer Materialien, beispielsweise Tantal, Titan, Edelstahl oder körperverträglicher Kunststoffe, beispielsweise Polyethylen, Polyamid oder Polyurethanelastomere erreicht werden. Weiterhin kann der Stent anstelle durch Erzeugung von Durchbrechungen in einem röhrenförmigen Körper auch beispielsweise durch Verflechtungen von einzelnen, drahtähnlichen Elementen erzeugt werden.

## Patentansprüche

1. Stent zur transluminalen Implantation in Hohlorgane, insbesondere in Blutgefäße, Harnleiter, Speiseröhren oder Gallenwege, mit einem im wesentlichen röhrenförmigen Körper (1), der eine Vielzahl von Durchbrechungen (3, 4, 13, 14, 19) aufweist, die jeweils von langgestreckten Begrenzungselementen (5) begrenzt werden,
**dadurch gekennzeichnet,**
daß jeweils zumindest zwei in Umfangsrichtung des Stents benachbart angeordnete, dieselbe Durchbrechung (3, 4, 13, 14, 19) begrenzende Begrenzungselemente (5) über das Ende der Durchbrechung (3, 4, 13, 14, 19) hinaus zur Bildung eines einheitlichen Detektionselements (6, 12, 18) verlängert ausgebildet sind,
daß die Detektionselemente (6, 12, 18) zungenförmig ausgebildet sind und sich im wesentlichen in Längsrichtung des Stents erstrecken,
daß die Detektionselemente (6, 12, 18) in Umfangsrichtung des Stents eine größere Breite aufweisen als jedes der Begrenzungselemente (5) und
daß die Detektionselemente (6, 12, 18) zumindest an einem Ende des Stents vorgesehen sind.

2. Stent nach Anspruch 1,
dadurch **gekennzeichnet,**
daß im wesentlichen über den gesamten Umfang des Stents jeweils zwei benachbart angeordnete Begrenzungselemente (5) ein Detektionselement (12) bilden, wobei die Breite des Detektionselements (12) insbesondere im wesentlichen gleich der Breite zweier nebeneinander liegender Begrenzungselemente (5) ist.

3. Stent nach Anspruch 1,
dadurch **gekennzeichnet,**
daß in Umfangsrichtung alternierend jeweils zwei benachbart angeordnete Begrenzungselemente (5) ein Detektionselement (6) bilden, während die darauffolgenden zwei Begrenzungselemente (5) nicht über das Ende der von ihnen begrenzten Durchbrechung (3) hinaus verlängert sind, wobei die Breite des Detektionselements (6) insbesondere im wesentlichen gleich der Breite von vier nebeneinander liegenden Begrenzungselementen (5) ist.

4. Stent nach einem der Ansprüche 1 bis 3,
dadurch **gekennzeichnet,**
daß an beiden Enden des Stents Detektionselemente (6, 12, 18) vorgesehen sind.

5. Stent nach einem der vorhergehenden Ansprüche,
dadurch **gekennzeichnet,**
daß das Detektionselement (6, 12, 18) in Umfangsrichtung des Stents mindestens im wesentlichen doppelt so breit ausgebildet ist wie jedes der Begrenzungselemente (5).

6. Stent nach Anspruch 5,
dadurch **gekennzeichnet,**
daß das Detektionselement (12) in Umfangsrichtung des Stents im wesentlichen doppelt so breit ausgebildet ist wie jedes der Begrenzungselemente (5).

7. Stent nach einem der Ansprüche 1 bis 5,
dadurch **gekennzeichnet,**
daß das Detektionselement (6) in Umfangsrichtung des Stents im wesentlichen viermal so breit ausgebildet ist wie jedes der Begrenzungselemente (5).

8. Stent nach einem der vorhergehenden Ansprüche,
dadurch **gekennzeichnet,**
daß die freien Enden der Detektionselemente (6, 12, 18) keine scharfkantigen Abschnitte aufweisen und insbesondere abgerundet ausgebildet sind.

9. Stent nach einem der vorhergehenden Ansprüche,
dadurch **gekennzeichnet,**
daß die Länge der Detektionselemente (6, 12, 18) jeweils zwischen 25 und 200 %, insbesondere zwischen 35 und 150 %, bevorzugt zwischen 50 und 100 % der Länge einer Durchbrechung (3, 4, 13, 14, 19) beträgt.

10. Stent nach einem der vorhergehenden Ansprüche,
dadurch **gekennzeichnet,**
daß der röhrenförmige Körper (1) von einem komprimierten Zustand mit einem ersten Querschnittsdurchmesser in einen expandierten Zustand mit einem vergrößerten zweiten Querschnittsdurchmesser überführbar ist.

11. Stent nach Anspruch 10,
dadurch **gekennzeichnet,**
daß die Durchbrechungen (3, 4, 19) im komprimierten Zustand des Stents als schlitzförmige Öffnungen ausgebildet sind.

12. Stent nach Anspruch 10,
dadurch **gekennzeichnet,**
daß die die Expansion gewährleistenden Durchbrechungen (13, 14) sowohl im komprimierten als auch im expandierten Zustand als in Längsrichtung und in Umfangsrichtung des Stents ausgedehnte, flächige Durchbrechungen ausgebildet sind und daß im Bereich zwischen zwei in Längsrichtung des Stents benachbart angeordneten Durchbrechungen (13, 14) verstärkt ausgebildete Verbindungsstellen (15) vorgesehen sind.

13. Stent nach einem der vorhergehenden Ansprüche,
dadurch **gekennzeichnet,**
daß das zwischen den Durchbrechungen (3, 4, 13, 14, 19) liegende Material der Wand des röhrenförmigen Körpers (1) Umrandungselemente (20) für die Durchbrechungen (3, 4, 13, 14, 19) bildet, die in Längsrichtung des Stents miteinander verbunden und zur Expansion des Stents aufweitbar sind.

14. Stent nach Anspruch 13,
dadurch **gekennzeichnet,**
daß jedes Umrandungselement (20) aus zumindest zwei in Umfangsrichtung des Stents benachbart angeordneten, langgestreckten Begrenzungselementen (5) besteht, die an den Verbindungsstellen (15) miteinander verbunden sind.

15. Stent nach einem der Ansprüche 12 bis 14,
dadurch **gekennzeichnet,**
daß die verstärkt ausgebildeten Verbindungsstellen (15) durch sich in Umfangsrichtung des Stents erstreckende Verbreiterungen (16) der Begrenzungselemente (5) gebildet sind.

16. Stent nach einem der vorhergehenden Ansprüche,
dadurch **gekennzeichnet,**
daß die Durchbrechungen (13, 14) an ihren Enden (17) eine geringere Breite aufweisen als im Bereich zwischen den Enden (17).

17. Stent nach einem der Ansprüche 10 bis 16,
dadurch **gekennzeichnet,**
daß die Durchbrechungen (13, 14) im komprimierten Zustand des Stents an ihren Enden verjüngt verlaufend ausgebildet sind.

18. Stent nach einem der Ansprüche 15 bis 17,
dadurch **gekennzeichnet,**
daß die Wanddicke des röhrenförmigen Körpers (1) im wesentlichen gleich der Breite der Begrenzungselemente (5) in ihren unverbreiterten Bereichen ist.

19. Stent nach einem der vorhergehenden Ansprüche,
dadurch **gekennzeichnet,**
daß die Wanddicke des röhrenförmigen Körpers (1) zwischen 0,15 mm und 0,30 mm, insbesondere zwischen 0,18 mm und 0,27 mm, bevorzugt zwischen 0,2 mm und 0,24 mm beträgt.

20. Stent nach einem der Ansprüche 15 bis 19,
dadurch **gekennzeichnet,**
daß die Breite der Begrenzungselemente (5) in ihren unverbreiterten Bereichen zwischen 0,15 mm und 0,25 mm, insbesondere zwischen 0,18 mm und 0,22 mm, bevorzugt ca. 0,2 mm beträgt.

21. Stent nach einem der Ansprüche 15 bis 20,
dadurch **gekennzeichnet,**
daß die Breite der Begrenzungselemente (5) in ihren verbreiterten Bereichen zwischen 0,25 mm und 0,35 mm, insbesondere zwischen 0,28 mm und 0,32 mm, bevorzugt ca. 0,3 mm beträgt.

22. Stent nach einem der Ansprüche 12 bis 21,
dadurch **gekennzeichnet,**
daß das Detektionselement (18) in Umfangsrichtung des Stents im wesentlichen die Breite von zwei Begrenzungselementen (5) plus die Breite der von den Begrenzungselementen (5) begrenzten Durchbrechung (13, 14) besitzt.

## Claims

1. Stent for the transluminal implantation in hollow organs, in particular in blood vessels, ureters, oesophagi or gall tracts, comprising a substantially tubular body (1) which has a plurality of apertures (3, 4, 13, 14, 19) which are respectively bounded by elongate boundary elements (5), characterised in that in each case at least two boundary elements (5) which are arranged adjacent one another in the peripheral direction of the stent and bound the same aperture (3, 4, 13, 14, 19) are made so that they extend beyond the end of the aperture (3, 4, 13, 14, 19) and form a unitary detection element (6, 12, 18); in that the detection elements (6, 12, 18) are made tongue-like and extend substantially in the longitudinal direction of the stent; in that the detection elements (6, 12, 18) have a greater width in the circumferential direction of the stent than each of the boundary elements (5); and in that the detection elements (6, 12, 18) are provided at least at one end of the stent.

2. Stent in accordance with claim 1, characterised in that, over essentially the entire periphery of the stent, respective pairs of adjacently arranged boundary elements (5) each form a detection element (12) with the width of the detection element (12) being in particular substantially the same as the width of two boundary elements (5) lying alongside one another.

3. Stent in accordance with claim 1, characterised in that alternating pairs of adjacently disposed boundary elements (5) in the circumferential direction each form a detection element (6), whereas the following two boundary elements (5) are not extended beyond the end of the aperture (3) they bound, with the width of the detection element (6) being in particular the same as the width of four boundary elements (5) lying alongside one another.

4. Stent in accordance with one of the claims 1 to 3, characterised in that detection elements (6, 12, 18) are provided at both ends of the stent.

5. Stent in accordance with one of the preceding claims, characterised in that the detection element (6, 12, 18) is made at least substantially twice as wide in the circumferential direction of the stent as each of the boundary elements (5).

6. Stent in accordance with claim 5, characterised in that the detection element (12) is made substantially twice as wide in the circumferential direction of the stent as each of the boundary elements (5).

7. Stent in accordance with one of the claims 1 to 5, characterised in that the detection element (6) is made substantially four times as wide in the circumferential direction of the stent as each of the boundary elements (5).

8. Stent in accordance with one of the preceding claims, characterised in that the free ends of the detection elements (6, 12, 18) have no sharp-edged sections and are in particular of rounded shape.

9. Stent in accordance with one of the preceding claims, characterised in that the length of the detection elements (6, 12, 18) in each case amounts to between 25 and 200 %, in particular to between 35 and 150 %, and preferably to between 50 and 100 % of the length of an aperture (3, 4, 13, 14, 19).

10. Stent in accordance with one of the preceding claims, characterised in that the tubular body (1) is transferable from a compressed state with a first cross-sectional diameter into an expanded state with an enlarged, second, cross-sectional diameter.

11. Stent in accordance with claim 10, characterised in that the apertures (3, 4, 19) are made as slot-like openings in the compressed state of the stent.

12. Stent in accordance with claim 10, characterised in that the apertures (13, 14) which ensure the expansion are formed both in the compressed state and also in the expanded state as areal apertures which are extended in the longitudinal direction and in the circumferential direction of the stent; and in that connection locations (15) of thickened design are provided in the region between two apertures (13, 14) arranged adjacent to one another in the longitudinal direction of the stent.

13. Stent in accordance with one of the preceding claims, characterised in that the material of the wall of the tubular body (1) lying between the apertures 83, 4, 13, 14, 19) forms frame elements (20) for the apertures (3, 4, 13, 14, 19) which are connected together in the longitudinal direction of the stent and can be dilated for the expansion of the stent.

14. Stent in accordance with claim 13, characterised in that each frame element (20) comprises at least two elongate boundary elements (5) arranged adjacent to one another in the circumferential direction of the stent which are connected together at the connection locations (15).

15. Stent in accordance with one of the claims 12 to 14, characterised in that the connection locations (15) of reinforced design are formed by broadened regions (16) of the boundary elements (5) extending in the circumferential direction of the stent.

16. Stent in accordance with one of the preceding claims, characterised in that the apertures (13, 14) have a narrower width at their ends (17) than in the region between the ends (17).

17. Stent in accordance with one of the claims 10 to 16, characterised in that the apertures (13, 14) are formed so that they extend tapering towards their ends in the compressed state of the stent.

18. Stent in accordance with one of the claims 15 to 17, characterised in that the wall thickness of the tubular body (1) is at least substantially the same as the width of the boundary elements (5) in their non-broadened regions.

19. Stent in accordance with one of the preceding claims, characterised in that the wall thickness of the tubular body (1) amounts to between 0.15 mm and 0.30 mm, in particular to between 0.18 mm and 0.27 mm, and preferably to between 0.2 mm and 0.24 mm.

20. Stent in accordance with one of the preceding claims 15 to 19, characterised in that the width of the boundary elements (5) amounts in its non-broadened regions to between 0.15 mm and 0.25 mm, in particular to between 0.18 and 0.22 mm, and preferably to ca. 0.2 mm.

21. Stent in accordance with one of the claims 15 to 20, characterised in that the width of the boundary elements (5) in their broadened regions amounts to between 0.25 mm and 0.35 mm, in particular to between 0.28 and 0.32 mm, and preferably to ca. 0.3 mm.

22. Stent in accordance with one of the claims 12 to 21, characterised in that the detection element (18) has, in the circumferential direction of the stent, essentially the width of two boundary elements (5) plus the width of the aperture (13, 14) bounded by the boundary elements (5).

## Revendications

1. Stent pour l'implantation transluminale dans des organes creux, en particulier dans les vaisseaux sanguins, les conduits urinaires, les oesophages ou les voies biliaires, comprenant un corps essentiellement de forme tubulaire, qui présente une pluralité de traversées (3, 4, 13, 14, 19), lesquelles sont respectivement délimitées par des éléments de délimitation allongés (5),
caractérisé en ce que
au moins deux éléments de délimitation respectifs (5) agencés de manière voisine du stent en direction périphérique et délimitant la même traversée (3, 4, 13, 14, 19) sont réalisés de façon prolongée au-delà de l'extrémité de la traversée (3, 4, 13, 14, 19) pour former un élément de détection unitaire (6, 12, 18),
en ce que les éléments de détection (6, 12, 18) sont réalisés en forme de languette et s'étendent essentiellement en direction longitudinale du stent,
en ce que les éléments de détection (6, 12, 18) présentent en direction périphérique du stent une largeur plus importante que celle des éléments de délimitation (5), et
en ce que les éléments de détection (6, 12, 18) sont prévus à au moins une extrémité du stent.

2. Stent selon la revendication 1,
caractérisé en ce que
essentiellement sur la totalité de la périphérie du stent, deux éléments de délimitation agencés respectivement voisins (5) forment un élément de détection (12), la largeur de l'élément de détection (12) étant en particulier sensiblement égale à la largeur de deux éléments de délimitation (5) disposés côte à côte.

3. Stent selon la revendication 1,
caractérisé en ce que
deux éléments de délimitation (5) respectifs agencés de manière voisine en alternance en direction périphérique forment un élément de détection (6), tandis que les deux éléments de délimitation (5) qui suivent ne sont pas prolongés au-delà de l'extrémité de la traversée (3) qu'ils délimitent, et la largeur de l'élément de détection (6) est en particulier sensiblement égale à la largeur de quatre éléments de délimitation (5) disposés côte-à côte.

4. Stent selon l'une des revendications 1 à 3,
caractérisé en ce que
des éléments de détection (6, 12, 18) sont prévus aux deux extrémités du stent.

5. Stent selon l'une des revendications précédentes,
caractérisé en ce que
l'élément de détection (6, 12, 18) est réalisé en direction périphérique du stent au moins sensiblement deux fois aussi large que chacun des éléments de délimitation (5).

6. Stent selon la revendication 5,
caractérisé en ce que
l'élément de détection (12) est réalisé en direction périphérique du stent sensiblement deux fois aussi large que chacun des éléments de délimitation (5).

7. Stent selon l'une des revendications 1 à 5,
caractérisé en ce que
l'élément de détection (6) est réalisé en direction périphérique du stent sensiblement quatre fois aussi large que chacun des éléments de délimitation (5).

8. Stent selon l'une des revendications précédentes,
caractérisé en ce que
les extrémités libres des éléments de détection (6, 12, 18) ne présentent aucun tronçon à arêtes vives, et sont réalisées en particulier de façon arrondie.

9. Stent selon l'une des revendications précédentes,
caractérisé en ce que
la longueur des éléments de détection (6, 12, 18) s'élève respectivement entré 25 et 200%, en particulier entre 35 et 150%, de préférence entre 50 et 100% de la longueur d'une traversée (3, 4, 13, 14, 19).

10. Stent selon l'une des revendications précédentes,
caractérisé en ce que
le corps en forme de tube (1) peut être passé d'un état comprimé avec un premier diamètre transversal jusque dans un état étendu avec un second diamètre transversal augmenté.

11. Stent selon la revendication 10,
caractérisé en ce que
les traversées (3, 4, 19) sont réalisées sous forme d'ouvertures en forme de fentes dans l'état comprimé du stent.

12. Stent selon la revendication 10,
caractérisé en ce que
les traversées (13, 14) qui assurent l'expansion aussi bien dans l'état comprimé que dans l'état étendu sont réalisées sous forme de traversées présentant une certaine surface et allongées en direction longitudinale et en direction périphérique, et en ce qu'il est prévu dans la région entre deux traversées (13, 14) agencées de manière voisine en direction longitudinale du stent, des emplacements de jonction (15) réalisés de manière renforcée.

13. Stent selon l'une des revendications précédentes,
caractérisé en ce que
le matériau de la paroi du corps tubulaire (1) situé entre les traversées (3, 4, 13, 14, 19) forme des éléments d'encadrement (20) pour les traversées (3, 4, 13, 14, 19), qui sont reliés les uns aux autres dans la direction longitudinale du stent, et sont susceptibles de s'élargir pour l'expansion du stent.

14. Stent selon la revendication 13,
caractérisé en ce que
chaque élément d'encadrement (20) est constitué par au moins deux éléments de délimitation (5) agencés au voisinage dans la direction périphérique du stent et allongés, qui sont reliés l'un à l'autre au niveau des emplacements de jonction (15).

15. Stent selon l'une des revendications 12 à 14,
caractérisé en ce que
les emplacements de jonction (15) réalisés de manière renforcée sont formés par des élargissements (16) des éléments de délimitation (5), qui s'étendent dans la direction périphérique du stent.

16. Stent selon l'une des revendications précédentes,
caractérisé en ce que
les traversées (13, 14) présentent à leurs extrémités (17) une largeur plus faible que dans la région entre les extrémités (17).

17. Stent selon l'une des revendications 10 à 16,
caractérisé en ce que
les traversées (13, 14) sont réalisées de manière à s'étendre en rétrécissement au niveau de leurs extrémités dans l'état comprimé du stent.

18. Stent selon l'une des revendications 15 à 17,
caractérisé en ce que
l'épaisseur de paroi du corps tubulaire (1) est essentiellement égale à la largeur des éléments de délimitation (5) dans leur région non-élargie.

19. Stent selon l'une des revendications précédentes,
caractérisé en ce que
l'épaisseur de paroi du corps tubulaire (1) s'élève entre 0,15 mm et 0,30 mm, en particulier entre 0,18 mm et 0,27 mm, de préférence entre 0,2 mm et 0,24 mm.

20. Stent selon l'une des revendications 15 à 19,
caractérisé en ce que
la largeur des éléments de délimitation (5) dans leur région non-élargie, s'élève entre 0,15 mm et 0,25 mm, en particulier entre 0,18 mm et 0,22 mm, de préférence environ 0,2 mm.

21. Stent selon l'une des revendications 15 à 20,
caractérisé en ce que
la largeur des éléments de délimitation (5) dans leur région élargie s'élève entre 0,25 mm et 0,35 mm, en particulier entre 0,28 mm et 0,32 mm, de préférence environ 0,3 mm.

22. Stent selon l'une des revendications 12 à 21,
caractérisé en ce que
l'élément de détection (18) possède en direction périphérique du stent sensiblement la largeur de deux éléments de délimitation (5), plus la largeur de la traversée (13, 14) délimitée par les éléments de délimitation (5).
